# EUROPÄISCHE PATENTSCHRIFT

(11) **EP 1 807 696 B1**
(45) Veröffentlichungstag und Bekanntmachung des Hinweises auf die Patenterteilung: **09.01.2013**
(21) Anmeldenummer: 05801676.7
(22) Anmeldetag: 04.11.2005
(51) Int. Cl.: G01N 33/487, G01N 27/30, G01N 33/50

(54) **VORRICHTUNG UND VERFAHREN ZUR MESSUNG VON ZELLEIGENSCHAFTEN**
DEVICE AND METHOD FOR MEASURING CELL PROPERTIES
DISPOSITIF ET PROCEDE POUR MESURER LES PROPRIETES DE CELLULES

(30) Priorität: 05.11.2004 EP 04026252; 20.12.2004 EP 04030135
(43) Veröffentlichungstag der Anmeldung: 18.07.2007
(73) Patentinhaber: Universität Osnabrück, 49074 Osnabrück (DE)
(72) Erfinder: WAGNER, Richard, 49186 Bad Iburg (DE); GALL, Karsten, 27616 Lunestedt (DE); WIRTH, Andreas, 48291 Telgte (DE)
(74) Vertreter: von Kreisler Selting Werner
(86) Internationale Anmeldenummer: PCT/EP2005/055752
(87) Internationale Veröffentlichungsnummer: WO 2006/048447

(56) Entgegenhaltungen:
- DE-A1- 10 047 390
- US-A1- 2002 064 841
- US-A1- 2003 146 091
- SAMSONOV ANDREY V ET AL: "Effects of membrane potential and sphingolipid structures on fusion of Semliki Forest virus." JOURNAL OF VIROLOGY, Bd. 76, Nr. 24, Dezember 2002 (2002-12), Seiten 12691-12702, XP002333347 ISSN: 0022-538X
- BELLEMARE F ET AL: "Evidence from incorporation and patch-clamp experiments for a nonselective channel of large conductance at the luminal membrane of rabbit proximal tubule" CANADIAN JOURNAL OF PHYSIOLOGY AND PHARMACOLOGY, Bd. 74, Nr. 3, 1996, Seiten 265-272, XP002333348 ISSN: 0008-4212
- SIMON S M ET AL: "LARGE AQUEOUS CHANNELS IN MEMBRANE VESICLES DERIVED FROM THE ROUGH ENDOPLASMIC RETICULUM OF CANINE PANCREAS OR THE PLASMA MEMBRANE OF ESCHERICHIA-COLI" PROCEEDINGS OF THE NATIONAL ACADEMY OF SCIENCES OF THE UNITED STATES OF AMERICA, Bd. 86, Nr. 16, 1989, Seiten 6176-6180, XP002333349 ISSN: 0027-8424
- HANYU YOSHIRO ET AL: "Simultaneous measurement of spectroscopic and physiological signals from a planar bilayer system: Detecting voltage-dependent movement of a membrane-incorporated peptide" BIOCHEMISTRY, Bd. 37, Nr. 44, 3. November 1998 (1998-11-03), Seiten 15376-15382, XP002333350 ISSN: 0006-2960
- MAYER MICHAEL ET AL: "Microfabricated teflon membranes for low-noise recordings of ion channels in planar lipid bilayers." BIOPHYSICAL JOURNAL, Bd. 85, Nr. 4, Oktober 2003 (2003-10), Seiten 2684-2695, XP002333351 ISSN: 0006-3495
- HENKART P ET AL: "INTERACTION OF LYMPHOCYTES WITH LIPID BI LAYER MEMBRANES A MODEL FOR LYMPHOCYTE MEDIATED LYSIS OF TARGET CELLS", PROCEEDINGS OF THE NATIONAL ACADEMY OF SCIENCES OF THE UNITED STATES OF AMERICA, vol. 72, no. 7, 1975, pages 2789-2793, ISSN: 0027-8424

## Beschreibung

Die Erfindung betrifft Verfahren zur Erfassung von Eigenschaften von Zellen und Vesikeln, sowie Messvorrichtungen zur Verwendung in diesen Verfahren.

Zur Erfassung von elektrophysiologischen Eigenschaften von Zellen ist die Patch-Clamp-Technik gebräuchlich. Dabei wird eine Glaspipette (Durchmesser etwa 1 µm) mit einer Elektrolytlösung gefüllt und vorsichtig auf die Oberfläche einer Zelle aufgesetzt. Man kann dann nach Perforation der Membranflecken in der Patch-Pipette Ströme oder das Potential über die gesamte Zelloberfläche messen (whole-cell recording). Man kann aber auch, und das ist der eigentliche Vorteil der Patch-Clamp-Technik, Ströme durch einzelne Kanäle die sich in dem Membranfleck (patch) direkt unterhalb der Pipettenspitze befinden, messen. In einem Verfahren zur teilweisen Automatisierung dieser Technik wird eine Zelle auf eine kleinere Öffnung zwischen zwei übereinander liegenden Kammern gesaugt, wobei die Öffnung an die Zellgröße angepasst werden muss. Ist die Zelle so stabilisiert und die Öffnung dadurch abgedichtet, können elektrische Messungen solange erfolgen, wie die Verbindung Zelle-Öffnung für Ionen weitestgehend abgeschlossen ist (Seal). Der Abdichtwiderstand zwischen Zelle und Öffnungsrand liegt dabei im Bereich von 10⁹ Ω; man spricht von einem Gigaseal. Dieser für hohe Auflösung (pA - nA) absolut notwendige zeitlich stabile Gigaseal ist das Hauptproblem bei der Automatisierung von Patch-Clamp-Messungen. Mit der gängigen Technik, eine Zelle auf eine kleinere Öffnung zu saugen, werden Abdichtwiderstände in einem Bereich von 10⁷ bis 10⁸ Ω erzielt, die zudem zeitlich nicht stabil sind. Ein reproduzierbar hoher Abdichtwiderstand lässt sich nur im Einzelexperiment und dort nur an einer geringeren Anzahl von Membrantypen erzielen, was die Patch-Clamp Technik in ihrer Anwendbarkeit stark einschränkt. Für die moderne Wirkstoffforschung bedeutet dies, dass man trotz vielfältiger Automatisierungsversuche weiterhin auf bislang nicht standardisierte, vornehmlich fluoreszenz-basierte Verfahren zurückgreifen muss. Für die Zulassung von Medikamenten, bei der die pharmakologische Sicherheit höchste Priorität genießt, und die fast ausschließlich nur mit elektrophysiologischen Messtechniken geprüft werden dürfen, stellen sequenzielle Einzelmessungen dabei eine erhebliche Hürde dar. Ein Screening von potentiellen Wirkstoffen in großem Umfang ist mit diesem Verfahren nicht möglich. Optische Messungen lassen sich an diesen automatisierten Patch-Clamp-Anlagen ebenfalls nicht durchführen.

Eine weitere etablierte Technik zur Erfassung elektrophysiologischer Daten sind vertikale Lipiddoppelschichten, die zwischen zwei mit Elektrolyt gefüllten Kammern gespannt werden (Borisenko et al., 2003; Hinnah et al., 2002). Nach Fusion von Ionenkanälen oder Transporterproteinen in die Lipiddoppelschichten kann der durch die Proteine vermittelte Strom bis zur Einzelmolekülebene aufgelöst werden. Die etablierte klassische Lipiddoppelschichten-Technik ist nicht automatisierbar. Die Messung von Eigenschaften von Membranen intakter Zellen ist im Gegensatz zur Patch-Clamp-Technik nicht möglich. Die Technik ist daher nicht für die pharmakologische Wirkstoffprüfung geeignet und zugelassen, da dafür nur Messverfahren an ganzen Zellen eingesetzt werden.

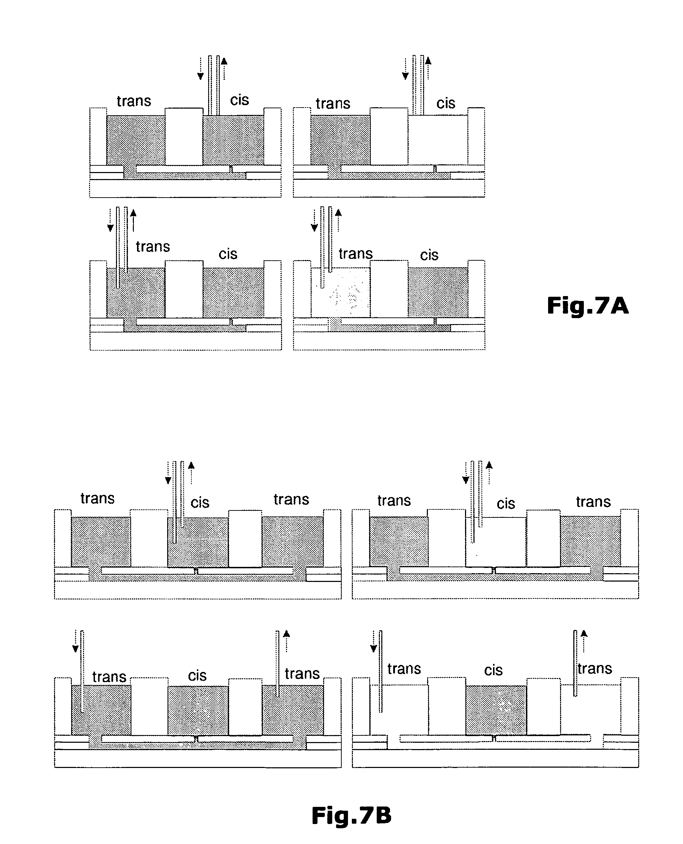

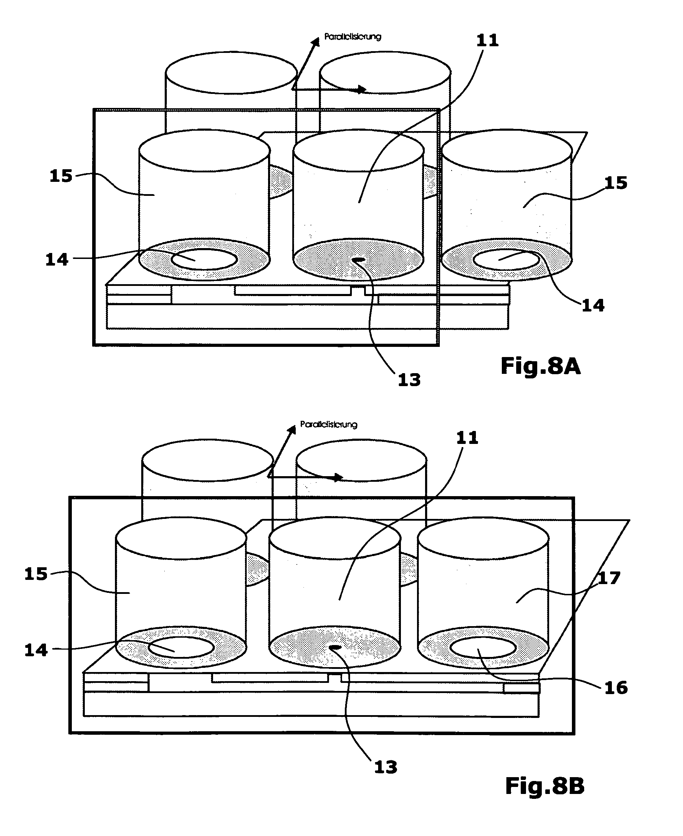

Die US 2003/0146091 A1 beschreibt eine Vorrichtung zur Durchführung von Messungen an Zellen mit einer oder mehreren Proben. In Ausführungsformen mit mehreren Messkammern (Figuren 7 und 8) wird dabei die Zelle an eine Öffnung gebunden, die in einer hydrophilen Trennwand, beispielsweise aus Siliciumnitrid oder Silica, positioniert ist. Die hydrophile Trennwand ist teilweise mit einer hydrophoben Beschichtung überzogen, damit die Zelle so positioniert werden kann, dass ihre Membran an die hydrophobe Beschichtung anschließt. Die mehrschichtige Vorrichtung ist vergleichsweise aufwändig herzustellen. Die Positionierung der Zellen auf der Vorrichtung ist störanfällig, da das Messobjekte selbst, also die Zellen, zunächst alle Öffnungen der Vorrichtung versiegeln müssen, um einen "Gigaseal" zu erreichen und somit Messungen vornehmen zu können.

Samsonov *et al.,* 2002, untersuchen die Fusion von Lipiddoppelschichten, die Fusionsfaktoren enthalten, mit Zellen, die Zellfusionsproteine an der Oberfläche expremieren. Dabei wird eine Messkammer verwendet mit einer hydrophoben Trennwand, die eine Öffnung von 150-200 µm enthält. In die Öffnung wird eine Lipiddoppelschicht eingefügt, die für die Fusion essentielle Faktoren wie Cholesterin und Sphingolipide enthält. Die Etablierung dieses Systems ist aufwändig, da die speziellen Fusionsfaktoren zugegeben bzw. expremiert werden müssen.

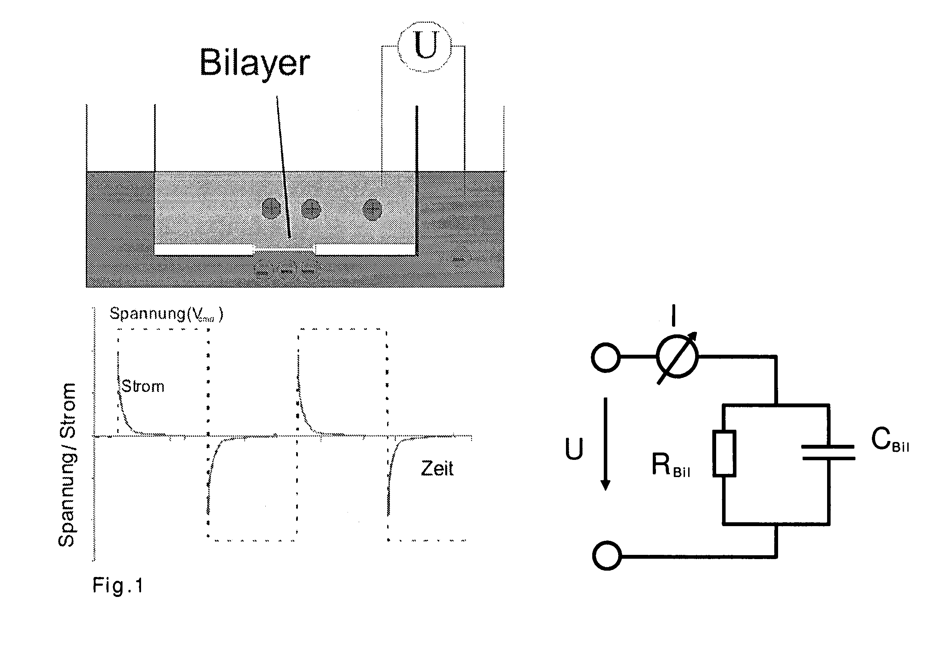

Die DE 10047390 A1 beschreibt Vorrichtungen mit einer Vielzahl von Messkammern, die jeweils durch eine Lipiddoppelschicht geteilt werden. Nach Anlegung einer Spannung durch die Lipiddoppelschichten können Veränderungen der Membranen bei Zugabe von organischen Verbindungen untersucht werden. Die Vorrichtung ist zum Hochdurchsatz-Screening geeignet. Allerdings weist die Vorrichtung Nachteile auf: So ist das Auseinandernehmen der Vorrichtung und das Aufbringen der Bilayer gemäß Abb. 1b umständlich. Die unteren Probenkammern sind nicht gut zugänglich und können nur schwer gleichmäßig befüllt und gereinigt werden. Die Silber-Elektroden sind so angeordnet, dass optische Messungen nicht möglich sind. Da die unteren Kammern nach Ausbildung der Lipiddoppelschicht vollständig abgeschlossen sind, kann bei Zugabe von Proben in die Kammer oberhalb der Membran ein osmotischer Druck entstehen, der die Membranen beeinträchtigt und dadurch die Messungen verfälscht.

Hanju et al., 1998 beschreiben Untersuchungen von vertikal angeordneten Lipiddoppelschichten in einer Einfachmesskammer. Eine Untersuchung von Zellen oder Vesikeln wird nicht durchgeführt und wäre wegen der Anordnung der Messkammer auch nur möglich, wenn diese durch besondere Hilfsmittel an die horizontale Membran fixiert würden.

Bellemare et al, 1996 untersuchen Ionenkanäle in Lipiddoppelschichten. Als Messanordung wird ein Delrin-Cup, also im wesentlichen ein kleineres Gefäß mit einer vertikalen Messöffnung in einem größeren Gefäß, verwendet, wobei die Öffnung einen Durchmesser von 200 bis 400 µm aufweist.

Sanford et al., 1989 beschreiben ebenfalls Untersuchungen von Lipiddoppelschichten in einer Einfachmesskammer an einer vertikalen Membran.

Aufgabe dieser Erfindung ist die Entwicklung einer neuartigen mikro- bzw. nanoanalytischen Technologie, die die Nachteile der oben beschriebenen Verfahren überwindet. Es sollen insbesondere ein Verfahren und Hilfsmittel bereitgestellt werden, um Zelleneigenschaften wie elektrophysiologische und optische Eigenschaften genau, zuverlässig und auf möglichst einfache und reproduzierbare Art zu erfassen. Das Verfahren soll automatisierbar und bereits im frühen Primärscreen von Wirkstoffen einsetzbar sein.

Die US-A-2002/0064841 offenbart Ionenelektroden, insbesondere Mikroelektroden und Elektrodenarrays sowie Verfahren zur Herstellung solcher Elektroden. Es werden planare Polymerelektroden für "patch clamp"-Messungen von Ionenströmen durch biologische Membranen, beispielsweise Plasmamembranen, lebender Zellen eingesetzt. Die dort beschriebenen Elektroden sind sinnvoll zur Messung individueller und mehrseitiger Zellmembranströme und -spannungen und können auch in "high-throughput screening"-Verfahren eingesetzt werden.

P. Henkart und R. Blumenthal berichten in Proc. Nat. Acad. Sci. USA, Vol. 72, No. 7, pp. 2789-2793, dass horizontal angeordnete Lipid-Doppelschichtmembranen als Modellsystem zum Studium von Lymphozytenvermittelten Eliminierung von Zielzellen eingesetzt wurden. LipidDoppelschichten, die dinitrophenyliert sind, können Dutzende von Lymphozyten stabil tragen, ohne zu brechen oder die elektrische Leitfähigkeit der Membran zu erhöhen. In Gegenwart von Antikörpern gegen Dnp verursachten humane Lymphozyten schnell eine Zunahme der Membranleitfähigkeit in mehreren Größenordnungen, ohne die Membran zu zerbrechen. Offenbart werden aber nur elektrophysiologische Experimente und keine Beobachtungen der Änderungen der Zellmembran mittels optischer Methoden. Mit den in dieser Arbeit offenbarten Messkammern ist dies auch prinzipiell nicht möglich.

Das der Erfindung zugrunde liegende Problem wird überraschenderweise gelöst durch Vorrichtungen und Verfahren nach einem der Ansprüche 1 bis 17.

Die Erfindung offenbart eine Messvorrichtung zur Erfassung von mindestens einer Eigenschaft von Zellen oder von Vesikeln, die von einer hydrophoben Trennwand, die eine horizontale Öffnung (Messöffnung) aufweist, in zwei Kammern aufgeteilt wird, die Elektrolytlösung enthalten, wobei die Öffnung von einer Lipiddoppelschicht geschlossen ist. Mindestens eine Zelle oder ein Vesikel kontaktiert die Lipiddoppelschicht.

Über die Öffnung wird eine Lipiddoppelschicht (Lipidbilayer, Bilayer) gespannt. Diese Lipiddoppelschicht bildet sich spontan, wenn ein biologisches, aufgereinigtes natürlich vorkommende oder synthetisches Lipid in geeigneter Form auf ein hydrophobes Substrat aufgebracht wird. Ferner dichtet die Lipiddoppelschicht die Öffnung und damit beide Kammern gegeneinander mit einem Widerstand um typischerweise 10¹⁰ Ω ab. Die Stabilität der Lipiddoppelschicht über die Zeit ist umgekehrt proportional zum Radius der Öffnung und liegt schon bei Löchern mit 30 µm Durchmesser im Bereich von Stunden bis Tagen (Hinnah et al., 2002).

Wird eine biologische Zelle mit der Lipiddoppelschicht in Kontakt gebracht, koppeln ihre elektrischen Eigenschaften. Das Kontaktieren erfolgt dadurch, dass die Lipiddoppelschicht horizontal angeordnet ist. Wird die Zelle oder das Vesikel dann auf oder über die Lipiddoppelschicht gegeben, so gerät es hauptsächlich durch die Schwerkraft in Kontakt mit der Lipiddoppelschicht. Die Kontaktierung kann jedoch auch anderweitig bewirkt oder verstärkt werden, zum Beispiel durch Bewegung der Messvorrichtung, der Elektrolytlösung und/oder durch Ausnutzung von attraktiven Wechselwirkungen zwischen der Lipiddoppelschicht und der Zelle bzw. dem Vesikel. Beispielsweise kann die Kontaktierung auch bewirkt werden oder verstärkt werden durch den Einsatz von geeigneten Rezeptor/Ligandensystemen oder Oberflächenproteinen, die die Zellfusion fördern. Solche gezielten Wechselwirkungen sind jedoch für das offenbarte System nicht erforderlich. Die Zell/Membranfusion wird nicht künstlich verstärkt durch den Zusatz entsprechender Fusionskomponenten oder durch vorherige gezielte Expression von Proteinkomponenten, die die Fusion bewirken oder fördern, wie beispielsweise die E1 und E2 Envelope Proteine. Vielmehr werden bereits alleine durch die Zugabe der Vesikel zu einer Lipiddoppelschicht Bedingungen geschaffen, die auf einfache Art die Messung der Vesikeleigenschaften erlauben.

Die molekularen Mechanismen der Kopplung sind im Einzelnen nicht bekannt, ihr Resultat ist jedoch eine Kopplung der Zelle mit der Lipidmembran, bei der die Zelle weitgehend ihre morphologische Struktur aufrecht erhält und zusammen mit dem Bilayer einen gemeinsamen elektrischen Widerstand darstellt (ohmsche Kopplung). Die Lipidmembran der Zelle oder des Vesikels fusioniert nach Kontaktierung mit der Lipiddoppelschicht teilweise oder vollständig mit ihr. Die Bestandteile der Zell- oder Vesikelmembran, z. B. die Ionenkanäle, werden dadurch Bestandteile der Lipidbilayerbarriere, die die Kammern trennt. Die Erfassung der Zelleigenschaften erfolgt vorzugsweise, wenn die Fusion abgeschlossen ist und sich das System stabilisiert hat.

Es ist jedoch zu betonen, dass das offenbarte Verfahren auch dann anwendbar ist, wenn eine Zelle oder ein Vesikel mit einer Lipiddoppelschicht in Kontakt steht, und beide eine gemeinsame Kapazität ausbilden (kapazitive Kopplung), ohne dass eine Fusion der Membranen auftritt. Bereits eine solche Anordnung erlaubt z. B. die Messung elektrischer Eigenschaften der Zelle, die bei Anlegung einer Spannung durch die Lipiddoppelschicht wie ein Kondensator fungiert.

Die horizontale Öffnung (Messöffnung) weist einen Durchmesser von 0,1 bis 100 µm, insbesondere 0,5 bis 50 oder 2 bis 40 µm auf. Die Öffnung ist vorzugsweise rund oder oval. Die Lipiddoppelschicht, die in der Öffnung positioniert ist, ist bedingt durch die Öffnung im wesentlichen horizontal. Dies bedeutet, dass sie leicht nach oben oder unten gewölbt sein kann. Eine solche Wölbung kann beispielsweise entstehen, wenn die Lösungen oberhalb und unterhalb der Membran unterschiedlich sind.

Die hydrophobe Trennwand kann, muss aber nicht horizontal sein. Sie kann beispielsweise ein leichte Neigung zur Messöffnung hin aufweisen, damit eine Zelle, die in die erste Kavität (11) gegeben wird, durch die Schwerkraft in Richtung zur Lipiddoppelschicht gelangt. Die Neigung der hydrophoben Trennwand zur Horizontale ist weniger als 45, 20 oder 10%.

Die hydrophobe Trennwand ist vorzugsweise eine Polymerfolie. In besonderen Ausführungsformen besteht die Trennwand aus Teflon, PMMA, PDMS, Topas oder Polycarbonat. Die Trennwand weist vorzugsweise eine Dicke von 1-100 µm auf. Geeignet sind allgemein Materialien, die ausreichend unpolar sind, so dass in einer Öffnung im Material eine Lipiddoppelschicht stabil eingebracht werden kann. "Hydrophob" bedeutet im Zusammenhang mit der Trennwand, dass in einer Öffnung in der Trennwand eine stabile Lipiddoppelschicht ausgebildet werden kann.

Die Elektrolytlösung ist vorzugsweise eine physiologische Salzlösung. Als Salze sind besonders solche geeignet, die die Ionen K⁺, Na⁺, Mg²⁺, Ca²⁺, Cl⁻, SO₄²⁻ und PO₄³⁻ enthalten. Der pH-Wert liegt bevorzugt zwischen 5 und 9 und wird mit geeigneten pH Puffersubstanzen eingestellt.

Die Lipiddoppelschicht besteht aus synthetischen und/oder aufgereinigten biologisch Lipiden. Die Erzeugung von Lipiddoppelschichten in Öffnungen in Trennwänden zur Durchführung von elektrophysiologischen Messungen ist nach dem Stand der Technik bekannt, beispielsweise aus Hinnah et al., 2002, auf das hier ausdrücklich Bezug genommen wird. Vorzugsweise wird zur Erzeugung der Lipiddoppelschicht eine Lipidpräparation verwendet aus synthetischen oder aufgereinigten biologlischen Lipiden. In einer solchen Lipidpräparation sind die Lipide nicht mehr in ihrer natürlichen Umgebung, also nicht mehr Bestandteile der intakten Zellorganelle, Zelle oder eines sonstigen natürlich vorkommenden Vesikels.

In einer bevorzugten Ausführungsform ist ein Schaltkreis durch die Messvorrichtung und durch die Lipiddoppelschicht angelegt, so dass Messungen bezüglich elektrischer Eigenschaften der Lipiddoppelschicht und der Zellen in Kontakt mit der Lipiddoppelschicht durchgeführt werden können.

In einer weiteren Ausführungsform besteht die Messvorrichtung zumindest teilweise aus optisch transparentem Material, so dass optische Messungen durchgeführt werden können. Beispielsweise kann untersucht werden, ob die Zelle bei Zugabe eines potentiellen Rezeptorliganden ein optisches Signal aussendet.

Gegenstand der Erfindung ist ein Verfahren zur Erfassung von Eigenschaften von Zellen oder Vesikeln unter Verwendung einer Messvorrichtung, die zwei durch eine hydrophobe Trennwand getrennte Kammern umfasst, wobei die Trennwand eine horizontale Öffnung aufweist, wobei
- in der Öffnung eine Lipiddoppelschicht positioniert wird, die die Öffnung abschließt,
- in beide Kammern eine physiologische Pufferlösung gegeben wird,
- mindestens eine Zelle in Kontakt mit einer Seite der Lipiddoppelschicht gebracht wird, und
- optische Eigenschaften gemessen werden.
Vorzugsweise ist die mindestens eine erfasste Eigenschaft der Zellen oder Vesikel eine optische oder elektrophysiologische Eigenschaft der Zellmembran. Erfindungsgemäß messbar sind beispielsweise der elektrische Widerstand der Membran, der Stromfluss durch die Membran und/oder Fluoreszenzsignale. Auf diese Art können nicht nur Eigenschaften der Zellmembran, sondern auch die Reaktion der Zellmembranen auf äußere Reize und Veränderungen, wie die Zugabe von Substanzen, untersucht werden. Es ist möglich, einzelne Werte punktuell zu ermitteln, oder die Veränderung von Parametern zu verfolgen und somit zelluläre Prozesse zu beobachten. Erfindungsgemäß kann zum Beispiel untersucht werden, wie durch die Zugabe eines (potentiellen) Rezeptorliganden zu der Messvorrichtung mit Lipiddoppelschicht und gekoppelter Zelle Ionenkanäle in der Zellmembran geöffnet oder geschlossen werden.

Nach der Fusionierung der Zelle mit der Lipiddoppelschicht sind außerdem Untersuchungen an beiden Seiten der Zellmembran möglich, je nachdem, in welche der beiden Kammern zum Beispiel Liganden gegeben werden.

Das erfindungsgemäße Verfahren ist insbesondere geeignet zur automatisierten Durchführung. Gegenstand der Erfindung ist auch eine Array zur automatisierten Erfassung von Eigenschaften von Zellen oder Vesikeln, bestehend aus mindestens zwei Messvorrichtungen. Die Messvorrichtungen sind dabei bevorzugt fest miteinander verbunden und elektrisch einzeln adressierbar. Die erfindungsgemäßen Arrays erlauben die Durchführung des Verfahrens zumindest teilweise durch Roboter oder Maschinen. Sie umfassen mindestens 2, bevorzugt 50 bis 2000, besonders 96, 384, oder 1536 Messvorrichtungen (SBS-Standard).

Die Erfindung erlaubt die Nutzbarmachung von mikroskopischen horizontalen Lipiddoppelschichten als Support für ganze Zellen. Diese in der Wirkstoffforschung wichtigen Untersuchungsobjekte lassen sich dabei nicht nur mit den bereits im High-Throughput-Screening (HTS) etablierten fluoreszenzbasierten Methoden analysieren, sondern simultan auch elektrisch.

Zudem lassen sich die einzelnen Support-Lipiddoppelschichten hochgradig miniaturisieren und zeitgleich oder zeitnah in nanotechnologisch strukturierten und gefertigten Arrays (Nanoarrays) vermessen. Durch die somit mögliche fast zeitgleiche Untersuchung verschiedener pharmakologisch relevanter Aspekte kann der Gesamtprozess der Wirkstofffindung deutlich verkürzt werden. Darüber hinaus wird die neue Technologie die Analyse von Membrantransportprozessen bedeutend erweitern. Durch diese Kopplung von Zelle und Lipiddoppelschicht können z. B. die elektrischen Eigenschaften einer nahezu beliebigen Zelle gemessen werden.

Auf diese Weise können reproduzierbar und mechanisch stabil die elektrischen und/oder optischen Eigenschaften der aufgebrachten Zellmembranen gemessen und charakterisiert werden. Im Gegensatz zur Patch- Clamp Technik werden bei dieser Erfindung keine Patch-Pipetten verwendet, die einen stabilen Abdichtwiderstand benötigen. Somit ist es auch nicht erforderlich, ein Vakuum oder einen Unterdruck anzulegen, um die Zelle an der Öffnung zu fixieren. Zwischen dem hydrophoben Substrat und der Lipiddoppelschicht bildet sich der Abdichtwiderstand spontan und mindestens über Stunden stabil aus. Ferner weist diese Verbindung zwischen Lipiddoppelschicht und hydrophober Folie die für HTS notwendige hohe mechanische Stabilität auf. Die Positionierung der Zellen auf der Lipiddoppelschift erfolgt bevorzugt einfach durch Zugabe dieser oberhalb der Lipiddoppelschicht.

Die hier beschriebene Methode ist zudem wesentlich kostengünstiger als die nach dem Stand der Technik bekannten beschriebenen, da die Messkammern parallel in Sandwich-Bauweise gefertigt werden können und keine mechanisch instabilen Bauteile wie Unterdruckleitungen enthalten.

Des Weiteren ist der optische Weg zur Zelle uneingeschränkt nutzbar, so dass sich diese Erfindung mit der hochauflösenden Fluoreszenzmikroskopie und -spektroskopie kombinieren lässt.

Die Kopplung zwischen Lipiddoppelschicht mit Zellen erlaubt es bei einem Abdichtwiderstand von beispielsweise 10⁸ - 10¹⁰ Ω die elektrischen Eigenschaften der Membranen zu messen. Zusätzlich erlaubt das Verfahren auch parallele optische Messungen. Eine genaue Positionierung der Zelle auf der Lipiddoppelschicht ist nicht notwendig, womit das Verfahren hochgradig automatisierbar und parallelisierbar ist.

Gegenstand der Erfindung sind auch Doppel- und Dreifachmesskammern sowie Mikrotiterplatten und Verfahren nach einem der Ansprüche 3 bis 8 und 10. Die erfindungsgemäßen Doppel- und Dreifach- Messkammern sind besonders geeignet zur Verwendung in einem erfindungsgemäßen Verfahren zur Bestimmung von Zelleigenschaften. Sie können aber auch allgemein zur Untersuchung von Eigenschaften von Lipiddoppelschichten ohne Kontaktierung mit Zellen verwendet werden.

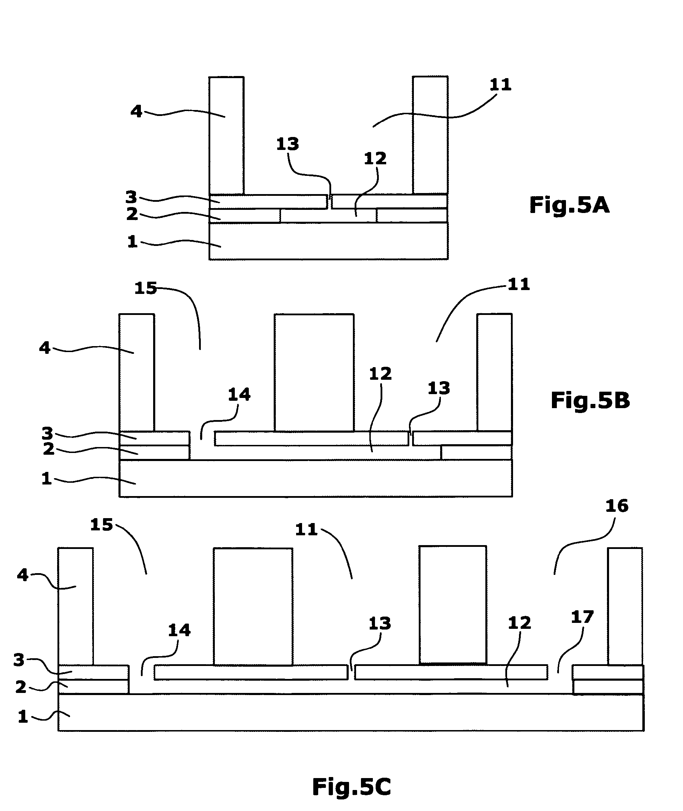

Figur 5a zeigt ein im erfindungmäßen Verfahrenverwendbares Einkammersystem mit einer ersten Kavität (11) mit seitlichen Wänden (4) und einer Trennwand (3), die gleichzeitig der Boden der Kavität ist. Unterhalb ist eine zweite Kavität (12) angeordnet, die mit der ersten Kavität (11) durch eine Messöffnung (13) verbunden ist. Die zweite Kavität (12) ist seitlich durch die Schicht (3) und durch einen unteren Boden (1) begrenzt. In beide Kavitäten werden Elektroden aus Metall positioniert. Der untere Boden besteht aus einem optisch aktiven Material wie Glas.

Figur 5b zeigt ein erfindungsgemäßes Doppelkammersystem mit zwei ersten Kavitäten (11) und (15) und einer durchgängigen zweiten Kavität (12), einer ersten Messöffnung (13) und einer zweiten Öffnung (14).

Figur 5c zeigt ein erfindungsgemäßes Dreikammersystem mit den Merkmalen gemäß Figur 5b. Zusätzlich ist eine weitere erste Kavität (16) enthalten mit einer Öffnung (17). Die zweite Kavität (12) erstreckt sich durchgängig unterhalb der ersten Kavitäten (11), (15) und (16).

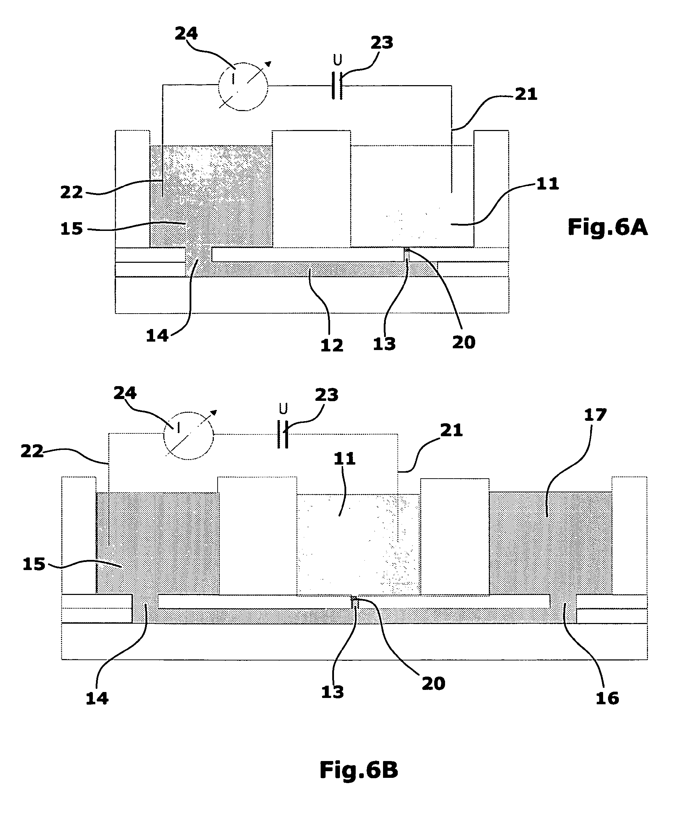

Figur 6a und b zeigen das erfindungsgemäße Doppel- bzw. Dreifachkammersystem mit den Merkmalen gemäß Figur 5b und 5c. Zusätzlich sind zwei Elektroden (21) und (22) enthalten, die über eine Spannungsquelle U (23) und ein Strommessgerät I (24) verbunden sind. In der Öffnung (13) ist eine Lipiddoppelschicht positioniert. Die Kavitäten sind mit Flüssigkeit befüllt, wobei die Lipiddoppelschicht zwei unterschiedlich zusammengesetzte Lösungen separiert.

Figur 7 zeigt schematisch, wie bei den erfindungsgemäßen Doppel- oder Dreifachkammersysteme mit Lipiddoppelschicht der Austausch von Flüssigkeit in bestimmten Kompartimenten erfolgen kann. Auf der linken Seite ist jeweils das System vor Austausch der Flüssigkeit gezeigt. Die Stellen der Entnahme und Zugabe sind durch Balken mit Pfeilen markiert. Auf der rechten Seite ist jeweils das System nach teilweisem Austausch der Flüssigkeit gezeigt, wobei die neu zugegebene Flüssigkeit in einem helleren Farbton dargestellt ist.

Figur 8 zeigt, wie die erfindungsgemäßen Doppel- oder Dreifachmesskammersysteme zu Arrays und Mikrotiterplatten angeordnet werden können. In Abbildung 8a ist eine Doppelmesskammereinheit durch Einrahmung gekennzeichnet, die mit analog angeordneten weiteren Doppelmesskammern über einen gemeinsamen Boden verbunden ist. Entsprechend ist in Figur 8b eine Dreifachmesskammer durch einen Rahmen gekennzeichnet.

Die offenbarte Einfachmesskammer, wie beispielhaft in Figur 5 dargestellt, umfasst eine erste Kavität (11) mit seitlichen Wänden (4) und einer Öffnung nach oben. Unter der ersten Kavität (11) ist eine zweite Kavität (12) positioniert, wobei die erste von der zweiten Kavität durch eine hydrophobe Trennwand (3) abgetrennt ist. Die hydrophobe Trennwand (3) weist eine Messöffnung (13) mit einem Durchmesser von 0,1 bis 100 µm auf. Mit "Messöffnung" wird hier eine Öffnung oder ein Loch bezeichnet, in das eine Lipiddoppelschicht eingebracht werden kann zur anschließenden Messung optischer oder elektrischer Eigenschaften in der Öffnung oder ihrer Umgebung. Dagegen dienen die Öffnungen (14, 17), die nicht als "Messöffnung" bezeichnet werden, nicht zur Aufnahme einer Lipiddoppelschicht, sondern ermöglichen den Flüssigkeitsaustausch und Stromfluß zwischen den ersten und zweiten Kavitäten. Sie weisen daher bevorzugt größere Durchmesser auf als die Messöffnungen.

In der ersten und zweiten Kavität (11, 12) ist je eine Elektrode aus Draht enthalten. Die Messkammer ist so beschaffen, dass in der Messöffnung (13) nach den oben beschriebenen Verfahren eine Lipiddoppelschicht positioniert werden kann, die die erste von der zweiten Kavität trennt. Über die beiden Elektroden kann eine Spannung durch die Lipiddoppelschicht angelegt werden. In die erste Kavität (11) können geeignete Flüssigkeiten wie Elektrolytlösungen und physiologische Puffersysteme oder zu untersuchende Zellen zugegeben werden.

Eine erfindungsgemäße Doppelmesskammer umfasst, wie in den Figuren 5B und 6A beispielhaft dargestellt, zwei nebeneinander angeordnete Messkammern, die jeweils eine erste Kavität (11, 15) mit seitlichen Wänden (4) und Öffnungen nach oben aufweist. Unter den beiden ersten Kavitäten (11, 15) ist eine durchgängige zweite Kavität (12) positioniert. Eine erste Kavität (15) ist mit der zweiten Kavität (12) durch eine Öffnung (14) verbunden. Die andere erste Kavität (11) ist von der zweiten Kavität (12) durch eine hydrophobe Trennwand (3) abgetrennt. Die Trennwand enthält (3) eine Messöffnung (13), die einen Durchmesser von 0,1 bis 100 µm aufweist, so dass die erste Kavität (11) mit der zweiten Kavität (12) verbunden ist. In den ersten Kavitäten (11) und (15) ist je eine Elektrode (21, 22) enthalten. Im Unterschied zur erfindungsgemäßen Einfachmesskammer ist in der zweiten Kavität (12) keine Elektrode vorgesehen. Die zweite Kavität (12) kann ausgestaltet sein wie ein Kanal und kann auf einfache Art befüllt und gespült werden. Die Anordnung der Doppelmesskammer ermöglicht einen Druckausgleich durch Veränderung der Flüssigkeitsmengen in den Kavitäten. Auf diese Art wird vermieden, dass die Lipiddoppelschicht beschädigt wird oder aufbricht. Die Möglichkeiten der Zugabe und Entnahme von Flüssigkeit sind schematisch in der Figur 7A dargestellt.

Eine erfindungsgemäße Dreifachmesskammer ist beispielhaft in den Figuren 5C und 6B gezeigt. Sie besteht aus einer Doppelmesskammer mit einer weiteren ersten Messkammer mit einer ersten Kavität (16), wobei unter den drei ersten Kavitäten (11, 15, 16) eine durchgängige zweite Kavität (12) vorhanden ist, und die erste Kavität (16) mit der zweiten Kavität (12) durch eine Öffnung (17) verbunden ist. Die Lipiddoppelschicht wird in der Messöffnung (13) positioniert, die die erste Kavität (11) von der zweiten Kavität (12) trennt.

Diese Anordnung hat den Vorteil, dass nach Ausbildung der Lipiddoppelschicht und damit nach Abtrennung der Kavität (11) ein durchgängiges Flüssigkeitssystem über die Kavitäten 12, 15 und 16 gegeben ist, über das ein Druckausgleich erfolgt. Darüber hinaus können die Flüssigkeit in der zweiten Kavität (12) in Anwesenheit der Lipiddoppelschicht ausgetauscht (gespült) werden. Dies ermöglicht zum Beispiel die kontinuierliche oder stufenweise Änderung der Ionenkonzentration, die Spülung und Reinigung der Kavität (12) oder die Zugabe oder Entfernung von Zusatzstoffen, deren Wirkung auf die Lipiddoppelschicht oder eine kontaktierende Zelle untersucht wird. In der Figur 7B ist schematisch gezeigt, wie in einem erfindungsgemäßen Dreifachkammersystem die Flüssigkeit in der mittleren ersten Kavität in Anwesenheit der Lipiddoppelschicht ausgetauscht werden kann. Bei der mittleren ersten Kavität (cis) erfolgt die Zugabe und Entnahme auf dem selben Weg, währen bei den beiden miteinander verbundenen ersten Kavitäten die Zugabe und Entnahme in unterschiedlichen Kavitäten erfolgen kann und der Druckausgleich über die untere Kavität erfolgt.

Durch den Druckausgleich wird auch verhindert, dass die Lipiddoppelschichten wegen des osmotischen Gefälles verändert oder sogar beschädigt werden und dadurch das Messergebnis verfälschen. Ganz allgemein ist es bei dem erfindungsgemäßen Vorrichtungen bevorzugt, dass ein Druckausgleich stattfindet, da die untere Kavität (12) mindestens eine Öffnung aufweist, die nicht durch einen Bilayer geschlossen ist.

Erfindungsgemäß können die Messkammern zu Mikrotiterplatten angeordnet werden. Die Mikrotiterplatten der vorliegenden Erfindung weisen mindestens zwei erfindungsgemäße Messkammern auf. Vorzugsweise sind die Messkammern identisch in zwei Raumrichtungen aneinander gereiht (Figur 8). Die Kammern enthalten in einer bevorzugten Ausführungsform einen optischen Zugang von unten, beispielsweise mittels einer Quarzglasscheibe, und einen Pipettierzugang von oben. Die Herstellung der Kammern kann in kostengünstiger Sandwichbauweise erfolgen. Dies gewährleistet einen Einwegansatz, wie er für pharmakologische Experimente benötigt wird. Mit den erfindungsgemäßen Vorrichtungen ist es möglich, unter Beibehaltung der SBS-konformen Mikrotiterplatten simultan und einzeln sowohl elektrische als auch optische Messungen in Hochdurchsatz an horizontalen Membranen durchzuführen. Durch parallelisierte Messkammern in zwei Raumrichtungen kann eine hohe Anzahl auf geringen Raum untergebracht werden kann.

Die in den Figuren dargestellten Messkammern sind lediglich schematisch dargestellt, insbesondere entsprechen in den Abbildungen die Größenverhältnisse der Bestandteile nicht unbedingt den realen Verhältnissen. Bevorzugt weist in einer Anordnung nach Figur 5 der Boden (1) einen vertikalen Durchmesser von 5-200, insbesondere 10-100 µm, die Schicht 2 einen Durchmesser von 5-200 µm, insbesondere 10-100 µm und die Trennwand (3) einen Durchmesser von 1-100 µm, insbesondere 5 bis 25 µm auf. Die Höhe der seitlichen Wände (4) ist vorzugsweise 0,5 bis 10, insbesondere 1 bis 5 mm und der horizontale Durchmesser der ersten Kavität 0,5 bis 10, insbesondere 1 bis 5 mm. Die ersten Kavitäten nehmen vorzugsweise 1 µl bis 1 ml Flüssigkeit auf. Die zweite Kavität (12) weist vorzugsweise den gleichen vertikalen Durchmesser auf wie die Schicht 2. Die Breite der zweiten Kavität kann variieren, vorzugsweise zwischen 0,1 und 500 µm.

Messungen der elektrophysiologischen Eigenschaften von "Membran-Sonden" und Membranproteinen, insbesondere Membrantransportern, gewinnen bei pharmakologischen Untersuchungen zunehmend an Bedeutung. Insbesondere ist es von Interesse, die elektrischen Eigenschaften der Membranproteine mit ihren optischen abzugleichen. Nach dem Stand der Technik ist zur Untersuchung von Lipiddoppelschichten die Methode von Borisenko et al. (2003) bekannt. Dabei ist auf der Unterseite einer hydrophoben Folie eine dünne Leiterschicht (z.B. Silber) aufgedampft, die den optischen Zugang frei lässt und im Falle elektrischer Messungen als Elektrode dient. Sind nur optische Messungen vorgesehen, kann auf die Silberschicht verzichtet werden. Die Messkammer hat die Nachteile, dass die Silberschicht optische Messungen beeinträchtigt und dass die vorhandene Agaroseschicht relativ aufwendig herzustellen ist und zudem in einem Zeitraum von Stunden durch Wasserverlust degeneriert. Die Vorrichtung ist daher insbesondere als Basis für ein automatisiertes Verfahren nicht geeignet. Mikrotiterplatten mit Volumina im Mikroliter-Maßstab (Mikrotiterplatten) werden in den gängigen Hochdurchsatz (HTS) und Ultra- Hochdurchsatz (UHTS)-Versuchsreihen der Wirkstoffsuche in der Pharmaforschung benötigt. Für bekannte elektrische und gekoppelte elektro-optische Messmethoden fehlt es an HTS- und UHTStauglichen Messkammern nach SBS-Standard (Society for Biomolecular Screening).

Die Doppel- und Dreifachmesskammer sowie Mikrotiterplatten der Erfindung sind besonders geeignet zur Erfassung von Eigenschaften von Zellen nach den erfindungsgemäßen Verfahren und zur Herstellung der erfindungsgemäßen Messvorrichtungen, bei denen mindestens eine Zelle die Lipiddoppelschicht kontaktiert. Die Ausführungsformen der erfindungsgemäßen Messvorrichtungen mit Zellen sind daher insbesondere Merkmale der Doppel- oder Dreifachmesskammern der Erfindung.

Die erfindungsgemäßen Messkammern und Mikrotiterplaten weisen gegenüber den bekannten Vorrichtungen zur Untersuchung von Lipiddoppelschichten zahlreiche Vorteile auf. Die erfindungsgemäßen Messkammern erlauben es, bei einem erfindungsgemäßen Verfahren Vorgänge zwischen zwei wässrigen Phasen, die durch eine horizontale Lipiddoppelschicht voneinander getrennt sind, optisch und/oder elektrisch zu verfolgen. Es wird die Verwendung miniaturisierter Messkammern sowie die Verwendung eines Sandwich-Aufbaus, bei dem eine hohe Anzahl von Messkammern mit einer geringen Anzahl von Bauteilen hergestellt werden, ermöglicht. Ein optischer Zugang ist durch den Boden der Vorrichtung gewährleistet. Der Pipetten- und Elektrodenzugang ist von oben gewährleistet, so dass das Messverfahren auf einfache Art und auch automatisiert durchgeführt werden kann.

Mit Hilfe von gängigen SBS- konformen Mikrotiterplatten lassen sich im Wesentlichen nur Veränderungen im Lumen einer Kammer mit optischen Methoden beobachten. Die erfindungsgemäße Mikrotiterplatte bietet dagegen die Möglichkeit, in zwei voneinander durch eine horizontale Lipiddoppelschichtmembran getrennten wässrigen Kompartimenten simultan oder einzeln optische und elektrische Messungen durchzuführen. Damit lassen sich die elektrischen und optischen Eigenschaften in den verschiedenen Kavitäten einzeln erfassen. Weiterhin ist es möglich, Transportvorgänge über die Membran sowie Vorgänge, die mit der Assoziation an die Membran, Dissoziation oder der Inkorporation von Effektoren in die Membran verbunden sind, ebenfalls optisch und elektrisch zu messen. Im Gegensatz zu herkömmlichen Mikrotiterplatten bieten die erfindungsgemäßen Mikrotiterplatten die Möglichkeit, Eigenschaften einer Membran oder Membranprozesse elektrisch und/oder optisch zu messen, ohne den Charakter einer UHTS Messkammerplatte zu verlieren.

Bei den erfindungsgemäßen Vorrichtungen werden mindestens zwei Kavitäten durch eine Lipiddoppelschicht getrennt. So können die elektrischen Parameter von Transportvorgängen über diese Membran gemessen werden. Um einen optischen Zugang zu gewährleisten, sollte dieser Bilayer ausreichend nah über der Glasplatte, d.h. innerhalb der Fokustiefe stabilisiert werden. Der Aufbau entspricht vorzugsweise einer Sandwichbauweise aus verschiedenen geeigneten Materialien wie z.B. Teflon und Glas. Ein solcher Schichtaufbau ist beispielsweise in der Figur 1 gezeigt (Schichten 1, 2, 3 und 4).

Der Aufbau mit nebeneinander liegenden Messkammern zu einer Doppel- oder Dreifachmesskammer reduziert die ursprüngliche Messkammeranzahl mindesten um den Faktor 2 (d.h. aus einer 96' er Mikrotiterplatte wird eine 48' er Doppelwellplatte, etc.). Dieser Mehrfachkammeraufbau hat jedoch den Vorteil, dass nach Trennung der Kammersysteme über eine horizontale Lipiddoppelschicht der Druckausgleich zwischen den Kammernsystemen erfolgen kann, ohne das sich die Lipiddoppelschicht verformt (Figur 7). Dies ist essenziell für das Arbeiten mit Ionengradienten zwischen beiden Kammern.

Eine bevorzugte Ausführungsform für eine erfindungsgemäße Doppel- und Dreifachmesskammer ist der Figur 5 zu entnehmen. Zwischen dem oberen Teil der Messkammern und dem Glasboden ist beispielsweise eine (oder mehrere) mikrostrukturierte Folie eingebracht. Durch diese werden mindestens zwei nebeneinander liegende Kammern zu einer Doppelkammer (Dreifachkammer etc., Mikrotiterplatte) verbunden. Pro Doppelkammer wird in die Folie ein Loch mit kleinem Durchmesser (0,1-100 µm) sowie ein Loch mit vorzugsweise größerem Durchmesser und eine horizontal beide Löcher verbindende Kavität (Kanal) eingebracht. Diese Kavität kann zum Beispiel in der Folie oder im Quarzglas integriert sein. In einer bevorzugten Ausführungsform ist bei einer Mikrotiterplatte die trennende Membran horizontal innerhalb der Fokustiefe über der Glasschicht angebracht.

Der erfindungsgemäße Einzelmesskammeraufbau mit zwei übereinander liegenden Kavitäten reduziert die Kammeranzahl bei Anordnung zu einer Mikrotiterplatte nicht. Er sollte aus Stabilitätsgründen vornehmlich bei symmetrischen oder ähnlichen Ionenkonzentrationen in beiden Kavitäten benutzt werden, da ein Druckausgleich zwischen den Kavitäten nur durch Verformen der Membran möglich ist.

Natürlich ist es auch möglich, weitere Messkammern mit den erfindungsgemäßen Dreifachmesskammern zu verbinden, um Systeme mit mindestens vier Messkammern zu erzeugen, die durch ein gemeinsames Kammer/Öffnungssystem miteinander in Verbindung stehen; solche Systeme sind als Dreikammersysteme mit zusätzlichen Messkammern Gegenstand der Erfindung.

### Ausführungsbeispiel:

In einem Kunststoffseptum aus einer 25 µm dicken Teflonfolie, das die beiden Kompartimente trennt, ist durch ein elektrisches Verfahren ein Loch mit etwa 30-50 µm Durchmesser erzeugt worden. In diesem Loch wird wie beschrieben (Hinnah et al., 2002) eine künstliche Lipiddoppelschicht zwischen den beiden wässrigen Kompartimenten erzeugt. Beide Kompartimente sind durch Ag⁺/AgCl-Elektroden elektrisch kontaktiert. Der Messaufbau der Kammer ist so dimensioniert, dass der Bilayer im Fokus eines Epi-oder konfokalen Fluoreszenzmikroskops erfasst werden kann (Figur 1). Ein solcher Bilayer hat typischerweise einen Abdichtwiderstand von >5 GΩ und zeigt beim Anlegen eines Spannungs-Gates (V_{cmd}) die im Schema nach Figur 1 skizzierte Stromantwort, bei der nach dem Laden der Bilayerkapazität der mittlere Strom bei V_{cmd}=100 mV <20 pA ist.

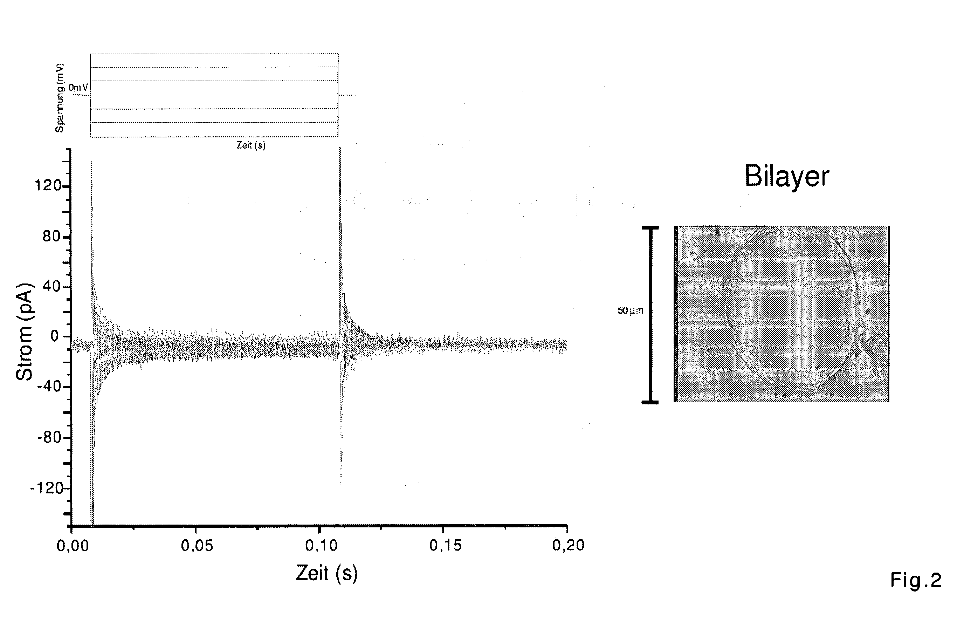

Figur 2 zeigt Stromableitungen ("Voltage Clamp") an einem horizontalen Bilayer (rechtes Bild) nach Anlegen von Spannungspulsen ("Spannungs-Gate", Bild links oben). Die Amplitude der Spannung (V_{cmd}) wurde in Inkrementen von V_{cmd}=+10mV ausgehend von V_{cmd}=0 mV bis V_{cmd} = 100 mV erhöht (Abbildung 2 links oben). Die untere linke Abbildung zeigt den durch die Kapazität und den Widerstand des Bilayers gewichteten zeitlichen Verlauf der Stromantwort. Dieser ist gekennzeichnet durch zwei kapazitive Peaks (*I* · *dt* = *C_{Bilayer}* · *V_{cmd})* und den konstanten *Strom* (*I* = *V_{cmd}*/*R_{Bilayer}*). In Figur 2 ist der reale Fall gezeigt, bei dem der Bilayer einen Durchmesser von 50 µm und einen mittleren Abdichtwiderstand von 9.5 GΩ hat.

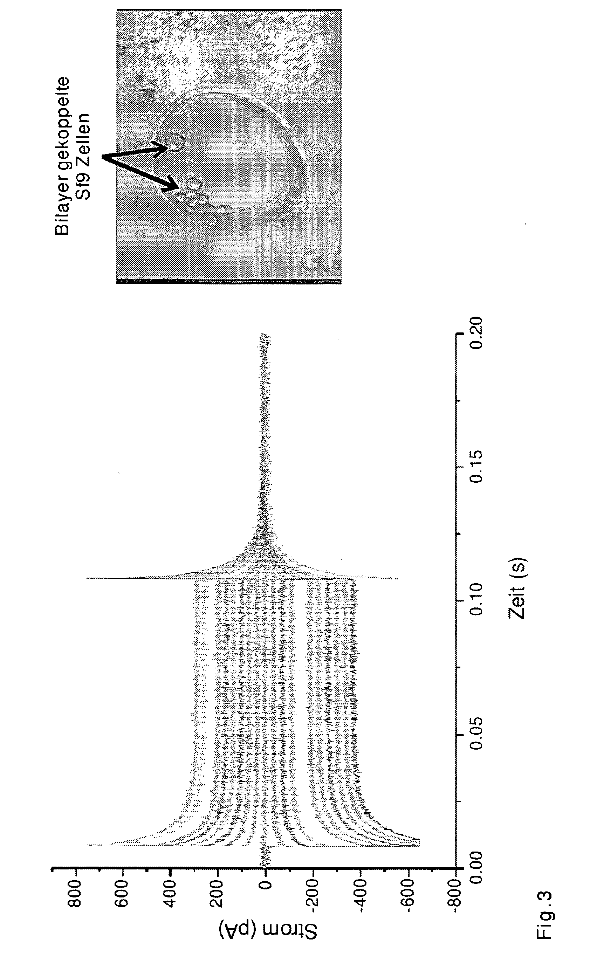

Auf diesen Bilayer wird mindestens eine SF-9 Zelle aufgebracht (Figur 3). Das Absetzen und die Manipulation einzelner oder mehrere Zellen erfolgt durch Mikropipetten und Mikromanipulatoren. Es wird dann nach einer erfolgten kapazitiven und ohmschen Kopplung die in Figur 3 gezeigte Stromantwort mit signifikant (> 40 mal verglichen mit Figur 2) größeren mittleren Strömen beim Anlegen von Spannungs-Gates gemessen. SF-9 Zellen sind eine aus Insekten (*Spodoptera Frugiperda*) abgeleitete Zelllinie, die für die heterologe Expression von Proteinen verwendet werden.

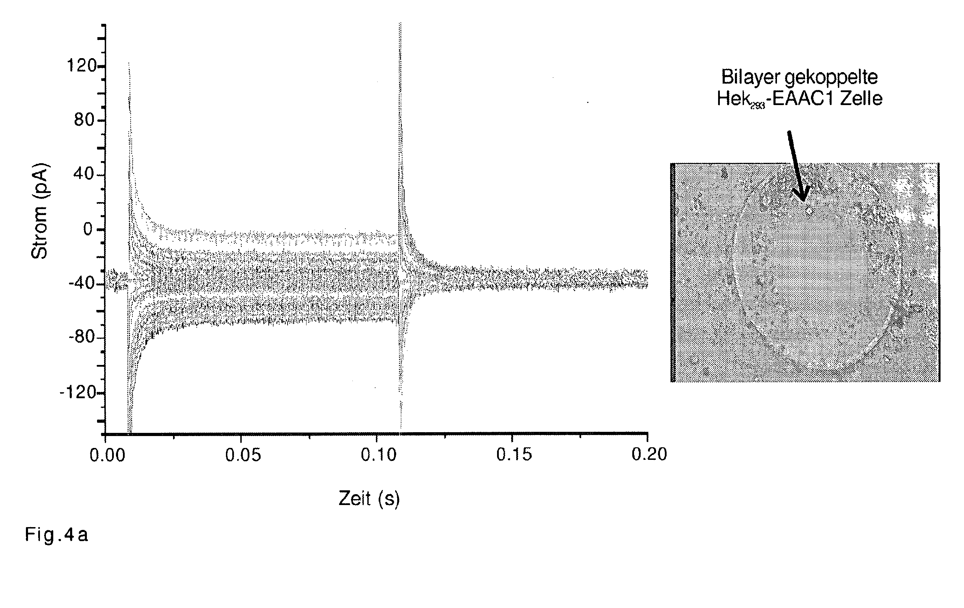

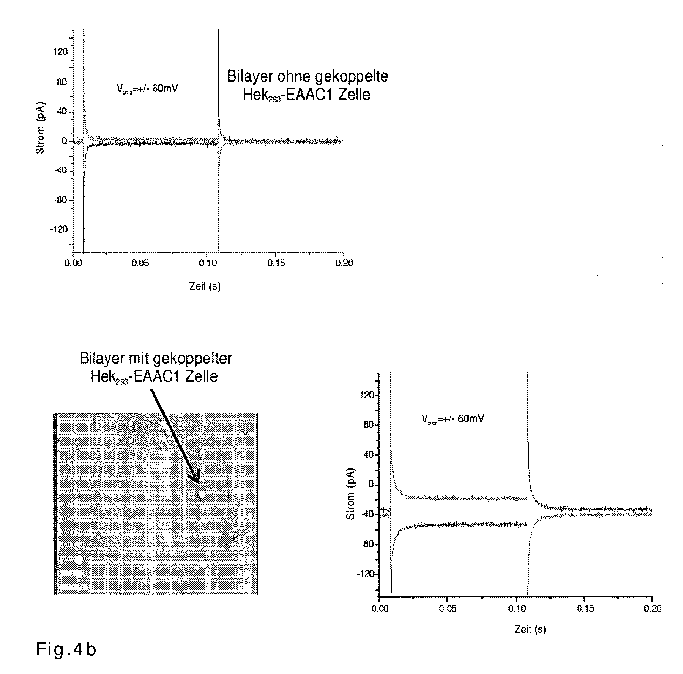

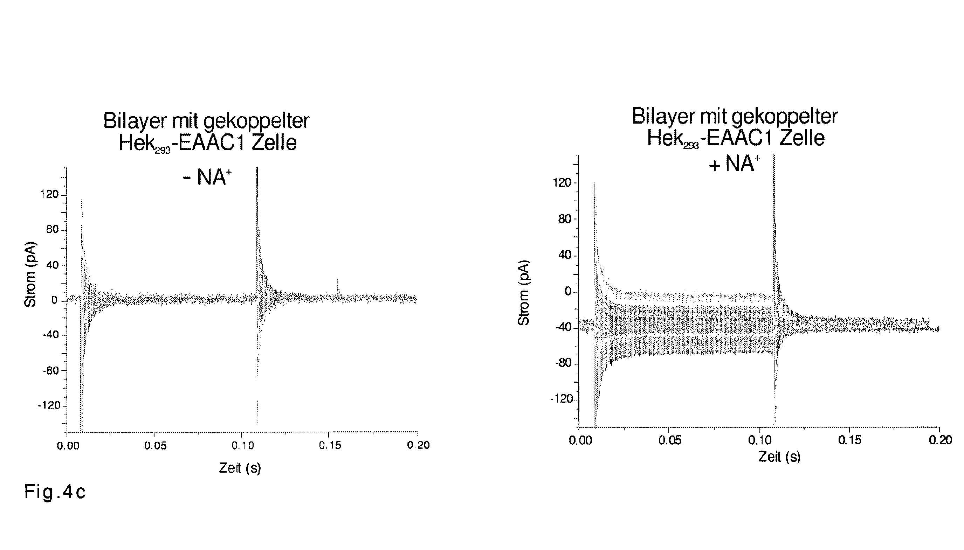

Die quantitative Verifizierung der Kopplung von Zellen auf einem horizontalen Bilayer anhand eines Na⁺-abhängigen Glutamattransporters (EAAC1), der heterolog in Hek-Zellen exprimiert wurde, ist in Figur 4 gezeigt. Figur 4a zeigt einen horizontalen Bilayer mit einer EAAC1-Hek₂₉₃-Zelle sowie die Stromantwort dieser Messanordnung. Eine quantitative Auswertung der Stromantwort bei V_{cmd} = ±60mV (Figur 4b) und ein Vergleich der mittleren Ströme mit den mittleren Strömen, die bei der gleichen Zellpräparation parallel in einer "Whole-Cell" Patch-Clamp-Konfiguration (Grewer et al., 2000) erhalten wurde, ist in Tabelle 1 gegenübergestellt.

**Tabelle 1**

| | **Patch Clamp** | **Bilayer** |
|---|---|---|
| | ganze Zelle Iₘᵢₜₜₑₗ in pA | gekoppelte Hek₂₉₃-EAAC1 Zelle Iₘᵢₜₜₑₗ in pA |
| 0 mV | -45 | -35 |
| + 60 mV | -10 | -15 |
| - 60 mV | -115 | -56 |
| -NA⁺ | 0 | 0 |

Der Glutamattransporter ist in seiner Aktivität streng Na⁺-abhängig (Grewer et al., 2000). Nachdem in beiden wässrigen Kompartimenten die Na⁺-Ionen durch Perfusion entfernt wurden, geht der mittlere Strom auf den Wert von Bilayern ohne gekoppelte Zelle zurück (Figur 4c). Bei erneutem Zufügen von Na⁺-Ionen werden die mittleren Ströme der Ausgangsmessanordnung erreicht (Figur 4c).

Diese Daten zeigen, dass die elektrische Stromantwort, die mit der "Whole Cell" Patch-Clamp-Anordnung an Hek₂₉₃-EAAC1 Zellen erhalten wird, mit der aus horizontalen Bilayern mit darauf gekoppelter Zelle identisch sind. Die Schwankungen der mittleren Ströme (Iₘᵢₜₜₑₗ, Tabelle 1) ist auf die biochemisch belegten Schwankungen der Expressionsraten von EAAC1 in Hek293 Zellen zurückzuführen. Diese Ergebnisse belegen, dass die Hek₂₉₃-EAAC1 Zellen kapazitiv und ohmsch mit dem horizontalen Bilayer gekoppelt sind. Der Vorteil des Verfahrens liegt darin, dass in einem horizontalen Aufbau zwei wässrige Kompartimente schnell und reproduzierbar durch eine Lipid-Doppelschicht hochohmig (>5GΩ) voneinander getrennt werden können. Das Aufbringen einer oder mehrerer Zellen auf diesen Bilayer, sowie das Aufbringen oder die Fusion von Makromolekülen auf bzw. in diese Bilayer Membran erlaubt es durch optische und elektrische Messverfahren Transport und Bindung von geladenen und nicht geladenen Molekülen über, bzw. an die Zellmembran, sowie entsprechend über die Lipid Bilayer Membran zu untersuchen. Das Verfahren ist aufgrund seiner Eigenschaften in hohem Maße für parallele Hochdurchsatzmessungen geeignet.

### Literatu r:

Borisenko V, Lougheed T, Hesse J, Fureder-Kitzmuller E, Fertig N, Behrends JC, Woolley GA, Schutz GJ. (2003) Simultaneous optical and electrical recording of single gramicidin channels. Biophys J 84(1): 612-22
Grewer C, Watzke N, Wiessner M, Rauen T. (2000) Glutamate translocation of the neuronal glutamate transporter EAAC1 occurs within milliseconds. Proc Natl Acad Sci U S A. 97(17):9706-11
Hinnah, S. C., Wagner, R., Sveshnikova, N., Harrer, R., and Soll, J. (2002) The chloroplast protein import channel Toc75: pore properties and interaction with transit peptides, Biophys. J. 83, 899-911.
Samsonov, A. V., Chatterjee, P. K., Razinkov, V. I., Eng, C. H., Kielian, M., Cohen, F. S. (2002) Effects of membrane potential and sphingolipid structures on fusion of Semliki Forest Virus, J. Virol., 76, p12691-12702.
Hanyu, Y., Yamada, T., Matsumoto, G. (1998) Simultaneous measurement of spectroscopic and physiological signals from a planar bilayer system: Detecting voltage-dependent movement of a membraneincorporated peptide, Biochem., 37, p15376-15382.
Bellamare, F., Dube, L., Sauve, R., (1996) Evidence from incorporation and patch-clamp experiments for a non-selective channel of large conductance at the luminal membrane of rabbit proximal tubule, Can. J. Physiol. Pharmacol., 74, p265-272.
Sanford, M. S., Blobel, G., Zimmerberg, J. (1989) Large aqueous channels in membrane vesicles derived from the rough endoplasmic reticulum of canine pancreas or the plasma membrane of E. coli, Proc. Natl. Acad. Sci. USA, 86, 6176-6180.

## Patentansprüche

1. Verfahren zur Erfassung von Zelleigenschaften unter Verwendung einer Messvorrichtung, die zwei durch eine hydrophobe Trennwand getrennte Kammern umfasst, wobei die Trennwand eine horizontale Öffnung aufweist, wobei
• in der Öffnung eine Lipiddoppelschicht bestehend aus synthetischen und/oder aufgereinigten natürlichen Lipiden positioniert wird, die die Öffnung abschließt,
• in beide Kammern eine physiologische Pufferlösung gegeben wird,
• mindestens eine Zelle in Kontakt mit einer Seite der Lipiddoppelschicht gebracht wird, unter Ausbildung einer ohmschen oder einer kapazitiven Kopplung und ,
• Fluoreszenzsignale mittels eines optisch transparenten Bodens der Messvorrichtung gemessen werden und Elektroden in den Kammern angeordnet werden und elektrische Eigenschaften gemessen werden.

2. Verfahren nach Anspruch 1, **dadurch gekennzeichnet, dass** das Verfahren automatisiert durchgeführt wird.

3. Doppelmesskammer, umfassend zwei nebeneinander angeordnete Messkammern, die jeweils eine erste Kavität (11, 15) mit seitlichen Wänden (4) enthalten, **dadurch gekennzeichnet, dass**
• unter den beiden ersten Kavitäten (11, 15) eine durchgängige zweite Kavität (12) mit einem Boden (1) positioniert ist,
• die erste Kavität (15) mit der zweiten Kavität (12) durch eine Öffnung (14) verbunden ist
• die erste Kavität (11) von der zweiten Kavität (12) durch eine hydrophobe Trennwand (3) abgetrennt ist,
• die Trennwand (3) eine horizontale Messöffnung (13) enthält, die einen Durchmesser von 0,1 bis 100 µm aufweist, so dass die erste Kavität (11) mit der zweiten Kavität (12) verbunden ist und durch die Messöffnung (13) eine Lipiddoppelschicht bestehend aus synthetischen und/oder aufgereinigten natürlichen Lipiden gespannt ist,
• in den ersten Kavitäten (11) und (15) je eine Elektrode (21, 22) enthalten ist und
• der Boden (1) aus optisch transparentem Material besteht.

4. Dreifachmesskammer, bestehend aus einer Doppelmesskammer nach Anspruch 3 und zusätzlich einer dritten Messkammer mit einer ersten Kavität (16), wobei
• unter den ersten Kavitäten (11, 15, 16) eine durchgängige zweite Kavität (12) positioniert ist, und
• die erste Kavität (16) mit der zweiten Kavität durch eine Öffnung (17) verbunden ist.

5. Doppel- oder Dreifachmesskammer nach einem der Ansprüche 3 oder 4, **dadurch gekennzeichnet, dass** die Öffnung (14) oder (17) einen Durchmesser von 10 µm bis 5 mm aufweist.

6. Doppel- oder Dreifachmesskammer nach einem der Ansprüche 3 bis 5, **dadurch gekennzeichnet, dass** die hydrophobe Trennwand (3) eine Polymerfolie ist.

7. Doppel- oder Dreifachmesskammer nach einem der Ansprüche 3 bis 5, **dadurch gekennzeichnet, dass** die Trennwand (3) aus Teflon, Polycarbonat, PMMA, PDMS oder Topas besteht.

8. Doppel- oder Dreifachmesskammer nach einem der Ansprüche 3 bis 7, **dadurch gekennzeichnet, dass** ein Schaltkreis durch die Messvorrichtung angelegt ist.

9. Array zur automatisierten Erfassung von Eigenschaften von Zellen oder Vesikeln, bestehend aus mindestens zwei Messvorrichtungen nach einem der Ansprüche 3 bis 8.

10. Mikrotiterplatte, bestehend aus mindestens zwei Doppel- oder Dreifachmesskammern nach einem der Ansprüche 3 bis 8, wobei die zweiten Kavitäten (12) durchgängig verbunden sind.

11. Verfahren zur Erfassung von Eigenschaften von Zellen, Vesikeln oder Lipiddoppelschichten unter Verwendung einer Doppel- oder Dreifachmesskammer nach einem der Ansprüche 3 bis 8 oder einer Mikrotiterplatte nach Anspruch 10.

12. Verfahren nach Anspruch 11, **dadurch gekennzeichnet, dass** das Verfahren automatisiert durchgeführt wird.

## Claims

1. A process for establishing cell properties using a measuring device comprising two chambers separated by a hydrophobic partition wall, wherein said partition wall has a horizontal opening, wherein:
• a lipid bilayer consisting of synthetic and/or purified natural lipids is positioned within the opening and closes the opening;
• a physiological buffer solution is added to both chambers;
• at least one cell is brought into contact with one side of said lipid bilayer to form an ohmic or capacitive coupling; and
• fluorescence signals are measured through an optically transparent bottom of the measuring device, and electrodes are arranged in the chambers and electrical properties are measured.

2. The process according to claim 1, **characterized by** being performed as an automated process.

3. A double measuring chamber comprising two juxtaposed measuring chambers each of which has a first cavity (11, 15) with lateral walls (4), **characterized in that**:
• a continuous second cavity (12) with a bottom (1) is positioned below the two first cavities (11, 15);
• said first cavity (15) is connected with said second cavity (12) through an opening (14);
• the first cavity (11) is separated from the second cavity (12) by a hydrophobic partition wall (3);
• said partition wall (3) contains a measuring opening (13) having a diameter of from 0.1 to 100 µm, so that said first cavity (11) is connected with said second cavity (12), and a lipid bilayer consisting of synthetic and/or purified natural lipids is spanned across the measuring opening (13);
• an electrode (21, 22) is contained in each of the first cavities (11) and (15); and
• said bottom (1) is made from an optically transparent material.

4. A triple measuring chamber consisting of a double measuring chamber according to claim 3 and additionally a third measuring chamber having a first cavity (16), wherein:
• a continuous second cavity (12) is present below the first cavities (11, 15, 16); and
• the first cavity (16) is connected with the second cavity through an opening (17).

5. The double or triple measuring chamber according to either of claims 3 or 4, **characterized in that** said opening (14) or (17) has a diameter of from 10 µm to 5 mm.

6. The double or triple measuring chamber according to any of claims 3 to 5, **characterized in that** said hydrophobic partition wall (3) is a polymer sheet.

7. The double or triple measuring chamber according to any of claims 3 to 5, **characterized in that** said partition wall (3) is made of Teflon, polycarbonate, PMMA, PDMS or Topas.

8. The double or triple measuring chamber according to any of claims 3 to 7, **characterized in that** a circuit has been applied through the measuring device.

9. An array for automatically establishing properties of cells or vesicles, consisting of at least two measuring devices according to any of claims 3 to 8.

10. A microtitration plate consisting of at least two double or triple measuring chambers according to any of claims 3 to 8, wherein said second cavities (12) are interconnected.

11. A process for establishing properties of cells, vesicles or lipid bilayers using a double or triple measuring chamber according to any of claims 3 to 8 or a microtitration plate according to claim 10.

12. The process according to claim 11, **characterized by** being performed as an automated process.

## Revendications

1. Procédé pour détecter des propriétés de cellules au moyen d'un dispositif de mesure comprenant deux chambres séparées par une paroi de séparation hydrophobe, la paroi de séparation présentant une ouverture horizontale, dans lequel
- une bicouche lipidique composée de lipides synthétiques et/ou naturels purifiés est positionnée dans l'ouverture, laquelle bicouche ferme l'ouverture,
- une solution tampon physiologique est introduite dans les deux chambres,
- au moins une cellule est mise en contact avec un côté de la bicouche lipidique, pour former un couplage ohmique ou capacitif et
- des signaux fluorescents sont mesurés au moyen d'un fond optiquement transparent du dispositif de mesure et des électrodes sont disposées dans les chambres et des propriétés électriques sont mesurées.

2. Procédé selon la revendication 1, **caractérisé en ce que** le procédé est réalisé de manière automatique.

3. Chambre de mesure double, comprenant deux chambres de mesure disposées côte à côte, qui contiennent chacune une première cavité (11, 15) avec des parois latérales (4), **caractérisée en ce que**
- une deuxième cavité perméable (12) comprenant un fond (1) est positionnée sous les deux premières cavités (11, 15),
- la première cavité (15) est reliée à la deuxième cavité (12) par une ouverture (14),
- la première cavité (11) est séparée de la deuxième cavité (12) par une paroi de séparation hydrophobe (3),
- la paroi de séparation (3) contient une ouverture de mesure horizontale (13) qui présente un diamètre de 0,1 à 100 µm, de sorte que la première cavité (11) est reliée à la deuxième cavité (12) et qu'une bicouche lipidique composée de lipides synthétiques et/ou naturels purifiés est tendue à travers l'ouverture de mesure (13),
- une électrode (21, 22) est contenue dans chacune des premières cavités (11) et (15) et
- le fond (1) est composé d'un matériau optiquement transparent.

4. Chambre de mesure triple composée d'une chambre de mesure double selon la revendication 3 et en plus d'une troisième chambre de mesure comprenant une première cavité (16), dans laquelle
- une deuxième cavité perméable (12) est positionnée sous les premières cavités (11, 15, 16), et
- la première cavité (16) est reliée à la deuxième cavité par une ouverture (17).

5. Chambre de mesure double ou triple selon l'une quelconque des revendications 3 ou 4, **caractérisée en ce que** l'ouverture (14) ou (17) présente un diamètre de 10 µm à 5 mm.

6. Chambre de mesure double ou triple selon l'une quelconque des revendications 3 à 5, **caractérisée en ce que** la paroi de séparation hydrophobe (3) est un film polymère.

7. Chambre de mesure double ou triple selon l'une quelconque des revendications 3 à 5, **caractérisée en ce que** la paroi de séparation (3) est composée de Téflon, de polycarbonate, de PMMA, de PDMS ou de Topas.

8. Chambre de mesure double ou triple selon l'une quelconque des revendications 3 à 7, **caractérisée en ce qu'**un circuit est réalisé à travers le dispositif de mesure.

9. Réseau pour détecter automatiquement des propriétés de cellules ou de vésicules, composé d'au moins deux dispositifs de mesure selon l'une quelconque des revendications 3 à 8.

10. Plaque de microtitrage, composée d'au moins deux chambres de mesure double ou triple selon l'une quelconque des revendications 3 à 8, dans laquelle les deuxièmes cavités (12) sont reliées de manière perméable.

11. Procédé pour détecter des propriétés de cellules, de vésicules ou de bicouches lipidiques au moyen d'une chambre de mesure double ou triple selon l'une quelconque des revendications 3 à 8 ou d'une plaque de microtitrage selon la revendication 10.

12. Procédé selon la revendication 11, **caractérisé en ce que** le procédé est réalisé de manière automatique.
